(19) Europäisches Patentamt
European Patent Office
Office européen des brevets

(11) **EP 3 640 610 B1**

(12) **EUROPEAN PATENT SPECIFICATION**

(45) Date of publication and mention of the grant of the patent:
**13.12.2023 Bulletin 2023/50**

(21) Application number: **18818309.9**

(22) Date of filing: **08.06.2018**

(51) International Patent Classification (IPC):
**G01G 3/14** *(2006.01)*     **A61B 5/11** *(2006.01)*
**A61B 5/113** *(2006.01)*     **A61G 7/05** *(2006.01)*
**G01G 19/52** *(2006.01)*     **G01G 23/00** *(2006.01)*

(52) Cooperative Patent Classification (CPC):
**G01G 19/445; A61B 5/0816; A61B 5/1102;**
**A61B 5/1114; A61B 5/113; A61B 5/6891;**
**A61G 7/0527; G01G 23/01; G01G 23/06;**
A61B 2562/0252; A61B 2562/046

(86) International application number:
**PCT/JP2018/021959**

(87) International publication number:
**WO 2018/230449 (20.12.2018 Gazette 2018/51)**

(54) **LOAD DETECTOR, LOAD DETECTION SYSTEM, AND BIOLOGICAL STATE MONITORING SYSTEM**

LASTDETEKTOR, LASTDETEKTIONSSYSTEM UND SYSTEM ZUR ÜBERWACHUNG EINES BIOLOGISCHEN ZUSTANDS

DÉTECTEUR DE CHARGE, SYSTÈME DE DÉTECTION DE CHARGE ET SYSTÈME DE SURVEILLANCE D'ÉTAT BIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.06.2017   JP 2017119042**

(43) Date of publication of application:
**22.04.2020   Bulletin 2020/17**

(73) Proprietor: **Minebea Mitsumi Inc.**
**Kitasaku-gun, Nagano 389-0293 (JP)**

(72) Inventors:
• **OMORI, Kiyoshi**
  **Kitasaku-gun, Nagano 389-0293 (JP)**
• **IIDA, Norihito**
  **Kitasaku-gun, Nagano 389-0293 (JP)**
• **HAYASHIDA, Takahiro**
  **Kitasaku-gun, Nagano 389-0293 (JP)**
• **MASUDA, Shigemi**
  **Kitasaku-gun, Nagano 389-0293 (JP)**
• **TODOROKI, Shinsuke**
  **Kitasaku-gun, Nagano 389-0293 (JP)**

(74) Representative: **Dennemeyer & Associates S.A.**
**Postfach 70 04 25**
**81304 München (DE)**

(56) References cited:
EP-A1- 2 183 554        EP-A2- 0 838 659
EP-B1- 2 183 554        WO-A1-2007/066638
JP-A- 2001 033 300      JP-A- 2006 252 540
JP-A- 2006 302 266      JP-A- 2014 180 432
JP-A- 2017 003 564      JP-B1- 6 195 953
US-A1- 2009 151 081     US-B1- 7 282 652

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a load detector provided with a damper member, a load detection system including the load detector, and a biological state monitoring system including the load detector.

BACKGROUND ART

For the sites of medical treatment and caregiving, it is proposed to detect the load of a subject (a human subject, that is, a person being monitored) on a bed via a load detector, so as to find the biological state of the subject on the basis of the detected load. In particular, for example, it is proposed to determine whether or not the subject is present on the bed (on-bed determination) on the basis of the detected load, and/or to estimate the respiratory rate and heart rate of the subject.

Patent Literature 1 and Patent Literature 2 each disclose an apparatus which is configured to detect whether a subject is present on a bed on the basis of a load value detected by a load detector which is provided under the four legs of the bed, and a load detector included in the apparatus.

Patent Literature 3 and Patent Literature 4 issued to the present applicant each disclose that a load detector is provided under the four legs of a bed to find the position of the center of gravity of a subject of the bed from a load value detected by the load detector and, based on that, to determine the subject's respiratory rate, extending direction of the body axis, position of the head, and the like.

**Citation List**

Patent Literature 1: Japanese Patent No. 4652226, specification.
Patent Literature 2: Japanese Patent No. 4879620, specification.
Patent Literature 3: Japanese Patent No. 6105703, specification.
Patent Literature 4: Japanese Patent No. 6122188, specification.

US 7 282 652 B1 discloses a hospital bed having load cells at the corners thereof, with wheels mounted below the load cells. EP 2 183 554 A1 discloses a bed having load cells at the corners thereof, and having a support frame including lift cylinders which allow raising and lowering as well as inclining the bed frame. EP 0 838 659 A2 discloses a hospital bed having a frame including load cells at the four corners. An elastomeric mounting block 48 is secured to an end of the load cell. JP2006-302266 discloses a load detector for use in conjunction with a hospital bed.

SUMMARY

**Technical Problem**

When detecting the load of the subject on the bed with four load detectors to support the bed at four points, and finding the biological state and the like of the subject on the basis of the obtained detection value, it is desirable to perform a load detection with the load detectors at a precision as high as possible.

Having made a number of researches for the high-precision detection from the above point of view, the present inventors have observed that there are cases where slight errors occur randomly in the detection value from the four load detectors. Then, through those researches, the present inventors have found out that the errors were caused by the fact that the bed had reached into a three-point supported state (i.e., a state of the bed being supported by other objects such as a floor surface and the like via three points at which the bed is supported. Because a rigid body is most stable in the three-point supported state, it always attempts to realize the three-point supported state).

An object of the present invention is to provide load detectors and a load detecting system which are capable of detecting the load of a subject on a bed at a higher precision by way of restraining the bed from reaching into the three-point supported state.

Another object of the present invention is to provide a biological state monitoring system which is capable of detecting the load of a subject on a bed at a higher precision by way of restraining the bed from reaching into the three-point supported state and, based on that, fining a biological state of the subject at a higher precision.

**Solution to the Problem**

The problem is solved by a load detector according to the invention as defined in claim 1. According to a first aspect of the present invention, there is provided a load detector to be used in a biological state monitoring system, the biological state monitoring system being configured to determine a biological state of a subject based on an output of four load detectors provided with respect to a bed so as to detect a load of the subject on the bed via four points of the bed, the load detector including:

>   a base;
>   a load cell supported on the base in a cantilever manner to have a free end;
>   a placement part which is attached to the load cell on a side of the free end of the load cell and onto which the bed is to be placed; and
>   a damper member which is provided below the base and which is a sheet having elasticity,
>   wherein the damper member is provided to prevent a contact between the base

and an installation surface on which the load detector is installed, in a state that the bed is placed on the placement part.

In the load detector according to the first aspect, the four load detectors of the biological state monitoring system may be configured to be provided at four corners of the bed.

In the load detector according to the first aspect, the biological state monitoring system may be configured to determine a biological information of the subject based on all of load values of the subject detected by the four load detectors respectively.

In the load detector according to the first aspect, the biological state monitoring system may further include a center of gravity position calculating unit configured to calculate a center of gravity position of the subject based on all of the load values of the subject detected by the four load detectors respectively, and the biological state monitoring system may be configured to determine the biological state of the subject based on the center of gravity position of the subject calculated by the center of gravity position calculating unit.

In the load detector according to the first aspect, the damper member may be attached to the base in contact with a lower surface of the base.

In the load detector according to the first aspect, the base may include a first base and a second base; the load cell may include a first load cell which is supported on the first base in a cantilever manner to have a free end, and a second load cell which is disposed to face the first load cell and which is supported on the second base in a cantilever manner to have a free end; the placement part may further include a first connection part connected to the first load cell and a second connection part connected to the second load cell, and the placement part may be disposed between the first load cell and the second load cell; the free end of the first load cell and the free end of the second load cell may face opposite directions to each other in a front rear direction; and the first connection part of the placement part may be connected to the first load cell on a side of the free end of the first load cell and the second connection part of the placement part may be connected to the second load cell on a side of the free end of the second load cell, the load detector may further includes a plate attached to a lower surface of the first base and a lower surface of the second base, and wherein the damper member may be attached to a lower surface of the plate.

The load detector according to the first aspect may further includes a cover member which is provided below the base and which has an opening. In this case, the damper member may have a first part having a size in plan view (plain view, planer view) larger than a size in plan view of the opening, and a second part having a size in plan view smaller than the size in plan view of the opening, and the cover member and the base may be configured to sandwich the first part of the damper member in a state that the second part of the damper member protrudes downwardly via the opening.

According to a second aspect of the present invention, there is provided a load detecting system including:

>   four load detectors provided with respect to a bed so as to detect a load of the subject on the bed via four points of the bed; and
>   a load calculating unit configured to calculate the load of the subject based on an output of the four load detectors, wherein each of the four load detectors including:
>
>>   a base;
>>   a load cell supported on the base in a cantilever manner to have a free end;
>>   a placement part which is attached to the load cell on a side of the free end of the load cell and onto which the bed is to be placed; and
>>   a damper member which is provided below the base and which is a sheet having elasticity,
>>   wherein the damper member is provided to prevent a contact between the base and an installation surface on which the load detector is installed, in a state that the bed is placed on the placement part.

According to a third aspect of the present invention, there is provided a biological state monitoring system including:

four load detectors provided with respect to a bed so as to detect a load of the subject on the bed via four points of the bed; and
a biological state determining unit configured to determine a biological state of the subject based on an output of the four load detectors,
wherein each of the four load detectors including:

a base;
a load cell supported on the base in a cantilever manner to have a free end;
a placement part which is attached to the load cell on a side of the free end of the load cell and onto which the bed is to be placed; and
a damper member which is provided below the base and which is a sheet having elasticity,
wherein the damper member is provided to prevent a contact between the base and an installation surface on which the load detector is installed, in a state that the bed is placed on the placement part.

The biological state monitoring system according to the third aspect may further includes a center of gravity position calculating unit configured to calculate a center of gravity position of the subject based on the output from the four load detectors. In this case, the biological state determining unit may be configured to determine the biological state of the subject based on a temporal variation of the center of gravity position of the subject calculated by the center of gravity position calculating unit.

According to a fourth aspect of the present invention, there is provided a bed system including:

a bed; and
a biological state monitoring system of the third aspect,
wherein the four load detectors of the biological state monitoring system support the bed at four points.

**Effect of the invention**

The load detectors and the load detecting system of the present invention are capable of restraining the bed from reaching into the three-point supported state so as to detect the load of the subject on the bed at a higher precision.

The biological state monitoring system of the present invention is capable of detecting the load of the subject on the bed at a higher precision and, based on that, fining the biological state of the subject at a higher precision.

BRIEF DESCRIPTION OF THE DRAWINGS

Fig. 1 is a block diagram depicting a configuration of a biological state monitoring system according to an embodiment of the present invention.
Fig. 2 is an illustrative view for explaining an arrangement of load detectors for a bed.
Fig. 3 is an exploded perspective view of one load detector according to the embodiment of the present invention.
Fig. 4 is a perspective view of the load detector according to the embodiment of the present invention.
Fig. 5 is a flow chart depicting a method for monitoring a biological state of a subject by using the biological state monitoring system.
Fig. 6 is an illustrative view for an explanation on a comparative examination of comparing the effects of the load detectors according to a comparative example with the effects of the load detectors according to the embodiment of the present invention.
Figs. 7(a) and 7(b) are tables organizing a result about the load detectors according to the embodiment of the present invention, in the comparative examination explained with Fig. 6.
Figs. 8(a) and 8(b) are tables organizing a result about the load detectors according to the comparative example, in the comparative examination explained with Fig. 6.
Figs. 9(a) and 9(b) are graphs depicting an influence of the vibration of a floor surface on the detection values of the load detectors according to the embodiment of the present invention and the load detectors according to the comparative example, wherein Fig. 9(a) depicts the result when the frequency of the vibration of the floor surface is 0 to 100 Hz, and Fig. 9(b) is a partially enlarged graph of Fig. 9(a), depicting the result when the frequency of the vibration of the floor surface is 0 to 40 Hz.

Fig. 10(a) is a cross section view where first and second bases and damper members of the load detector of the embodiment of the present invention are cut up along a surface orthogonal to a front/rear direction of the load detector at the front/rear center.

Fig. 10(b) is a cross section view where the first and second bases and the damper members of the load detector of a first modified embodiment of the present invention are cut up along the surface orthogonal to the front/rear direction of the load detector at the front/rear center.

Fig. 10(c) is a cross section view where the first and second bases, a plate, and a damper member of the load detector of a second modified embodiment of the present invention are cut up along the surface orthogonal to the front/rear direction of the load detector at the front/rear center.

Fig. 10(d) is a cross section view where the first and second bases, a damper member, and a cover of the load detector of a third modified embodiment of the present invention are cut up along the surface orthogonal to the front/rear direction of the load detector at the front/rear center.

Fig. 10(e) is a cross section view where the first and second bases, a damper member, and the cover of the load detector of a fourth modified embodiment of the present invention are cut up along the surface orthogonal to the front/rear direction of the load detector at the front/rear center.

Fig. 11 is an exploded perspective view of a load detector according to the second modified embodiment of the present invention.

Fig. 12 is an exploded perspective view of a load detector according to the third modified embodiment of the present invention.

Fig. 13 is a block diagram depicting an overall configuration of a bed system according to the embodiment of the present invention.

## DESCRIPTION OF EMBODIMENT

### \<Embodiment\>

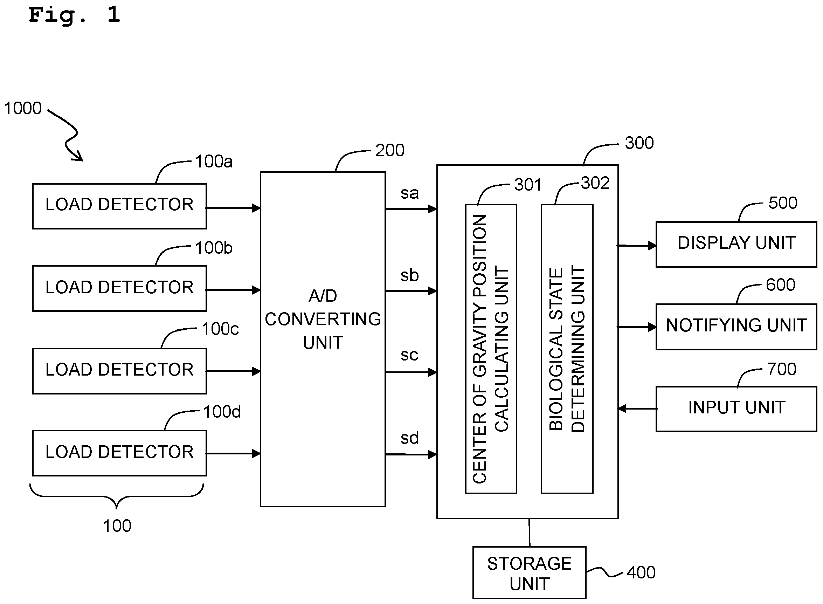

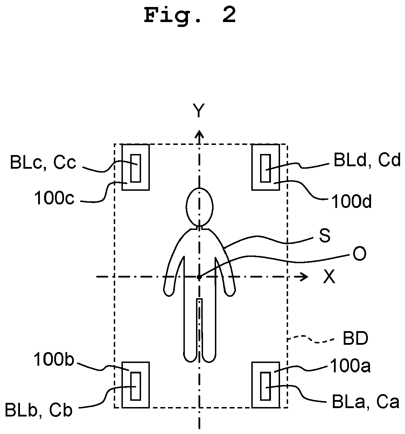

An explanation will be made on a biological state monitoring system 1000 (Fig. 1) according to an embodiment of the present invention, with an example of using the system with a bed BD (Fig. 2) to monitor a biological state (such as respiratory rate, heart rate, and the like) of a subject (human subject) S on the bed BD.

In the following explanation, with the center of the cuboid bed BD (Fig. 2) as the center O, the axis X of the bed BD is defined as the axis extending in the breadthwise (widthwise) direction and passing through the center O, and the axis Yof the bed BD is defined as the axis extending in the lengthwise (longitudinal or up/ down) direction and passing through the center O. In planar view of the bed BD, the positive side of the axis X is the right side of the center O of the bed BD whereas the negative side of the axis X is the left side thereof, and the positive side of the axis Yis the upper side of the center O of the bed BD whereas the negative side of the axis Yis the lower side thereof. When the subject S lies on the bed BD, generally he or she lies along the axis Y and the head is placed on the positive side and the feet are placed on the negative side in the axis-Y direction.

[Overall configuration of the biological state monitoring system]

As shown in Fig. 1, the biological state monitoring system 1000 primarily has a load detecting unit 100 and a control unit 300. The load detecting unit 100 and the control unit 300 are connected via an A/D converting unit 200. The control unit 300 is further connected to a storage unit 400, a display unit 500, a notifying unit 600, and an input unit 700.

The load detecting unit 100 includes four load detectors 100a, 100b, 100c, and 100d. The configuration of the load detectors 100a to 100d will be described later on. Each of the load detectors 100a to 100d is connected to the A/D converting unit 200 by way of wiring or wirelessly.

As shown in Fig. 2, the four load detectors 100a to 100d of the load detecting unit 100 are arranged respectively under casters Ca, Cb, Cc, and Cd fitted on the lower ends of legs BLa, BLb, BLc, and BLd at the four corners of the bed BD used by the subject S.

The A/D converting unit 200 includes an A/D convertor connected respectively to the load detecting unit 100 and the control unit 300 by way of wiring or wirelessly, to convert analog signals from the load detecting unit 100 to digital signals.

The control unit 300 is a dedicated or general-purpose computer inside which a center of gravity position calculating unit 301 and a biological state determining unit 302 are primarily constructed. The center of gravity position calculating unit 301 calculates the position of the center of gravity of the subject S on the basis of a load signal from the load detecting unit 100. The biological state determining unit 302 determines the biological state of the subject S on the basis of the position of the center of gravity of the subject S calculated by the center of gravity position calculating unit 301. Descriptions will be made later on about the calculation of the position of the center of gravity of the subject in the center of gravity position calculating unit 301, and the determination of the biological state of the subject in the biological state determining unit 302.

The storage unit 400 is a storage device storing data used in the biological state monitoring system 1000 and, for example, can use a hard disk (magnetic disk) for that purpose.

The display unit is a monitor such as a liquid crystal monitor or the like for displaying the biological state and the like of the subject outputted from the control unit 300 for a user of the biological state monitoring system 1000 (for example, healthcare professionals, caregiving professionals, and the like). The notifying unit 600 is provided with a device such as a speaker for example, for auditorily performing predetermined notifications on the basis of the output from the control unit 300 (information and the like about the biological state). The input unit 700 is an interface for performing predetermined inputs for the biological state monitoring system 1000, which may be a keyboard and a mouse.

[Configuration of the load detectors]

Next, configuration of the load detectors 100a to 100d will be explained. Because the load detectors 100a to 100d have an identical configuration to each other, the load detector 100a is selected here as the representative for the explanation with an exemplary case where the caster Ca of the leg BLa of the bed BD is fitted on the load detector 100a.

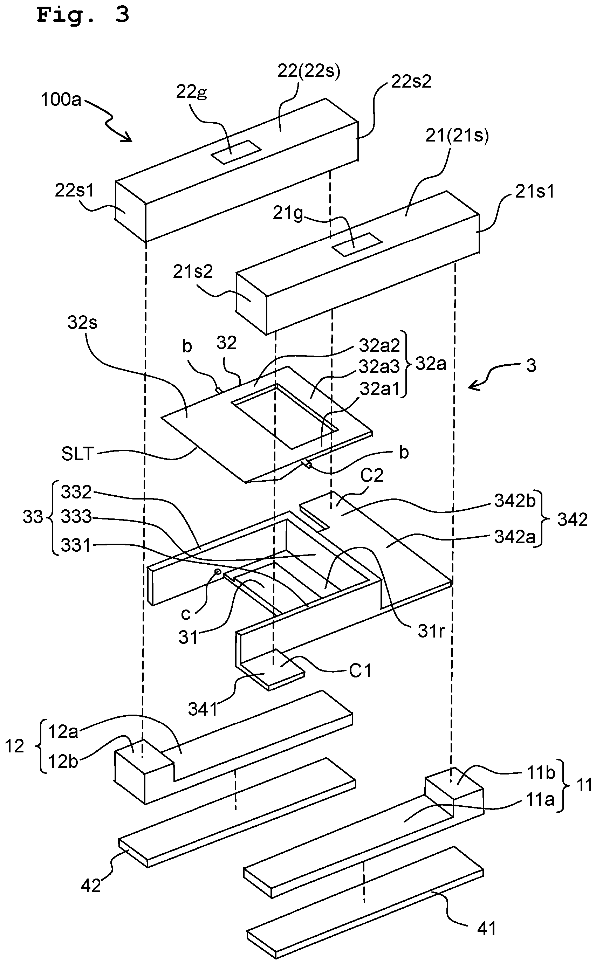

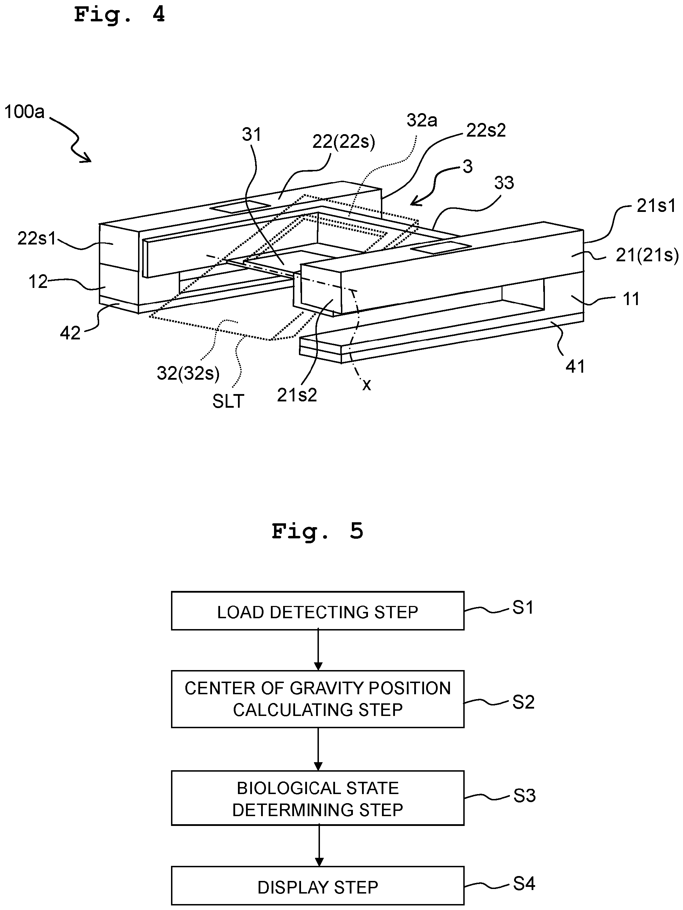

As shown in Figs. 3 and 4, the load detector 100a primarily includes first and second bases 11 and 12, first and second beam-type load cells 21 and 22 supported in a parallel and cantilevered manner by the first and second bases 11 and 12, a placement part 3 being supported by the first and second beam-type load cells 21 and 22 in a vertically slightly movable (displaceable) manner and having a movable slope 32, and damper members 41 and 42 provided on the lower surfaces of the first and second bases 11 and 12. Note that in Fig. 4, in order to clearly show a positional relation to other structures, the movable slope 32 is drawn with dotted lines for sight through.

In the explanation about the load detector 100a, the direction of the extending first and second beam-type load cells 21 and 22 is defined as a front/rear direction of the load detector 100a, the front side is the side where the leading end SLT of the movable slope 32 is positioned, and the rear side is the opposite side thereto. Further, the width direction of the load detector 100a is defined as a direction of the first and second beam-type load cells 21 and 22 opposing each other.

The first and second bases 11 and 12 have, respectively, elongate flat plates 11a and 12a extending in the front/rear direction, and support base parts 11b and 12b provided on the upper surfaces of the flat plates 11a and 12a. The support base part 11b of the first base 11 is provided at the rear end of the flat plate 11a whereas the support base part 12b of the second base 12 is provided at the front end of the flat plate 12a.

The first and second beam-type load cells 21 and 22 are arranged parallel and supported in a cantilevered manner (cantilever manner) by the first and second bases 11 and 12. The first and second beam-type load cells 21 and 22 have flexure elements 21s and 22s having an identical shape to each other, and strain gages 21g and 22g attached to the flexure elements 21s and 22s, respectively.

Each of the flexure elements (strain bodies) 21s and 22s is a Roberval-type flexure element formed of a metal such as aluminum, iron or the like, and shaped into a prism having a through hole (not shown) penetrating through in the width direction at a central part in the lengthwise direction of the prism. The strain gages 21g and 22g are attached to the upper surface and the lower surface of the flexure elements 21s and 22s in the vicinity of the through holes, one for each surface.

The flexure element 21s has its lower surface fixed on the support base part 11b of the first base 11, in the vicinity of its rear end. By virtue of this, with the rear end as a fixed end 21s1 and with the front end as a free end 21s2, the flexure element 21s is supported in a cantilevered manner by the first base 11 (the support base part 11b). On the other hand, the flexure element 22s has its lower surface fixed on the support base part 12b of the second base 12, in the vicinity of its front end. By virtue of this, with the front end as a fixed end 22s1 and with the rear end as a free end 22s2, the flexure element 22s is supported in a cantilevered manner by the second base 12 (the support base part 12b). That is, the free end 21s2 of the flexure element 21s and the free end 22s2 of the flexure element 22s face the opposite sides in the front/rear direction.

Further, the fixed end 21s1 and the free end 21s2 of the flexure element 21s are located at almost the same position as the free end 22s2 and the fixed end 22s1 of the flexure element 22s in the front/rear direction, respectively. Therefore, the support base part 11b of the first base 11 is located at almost the same position as the free end 22s2 of the flexure element 22s in the front/rear direction, whereas the support base part 12b of the second base 12 is located at almost the same position as the free end 21s2 of the flexure element 21s.

The placement part 3 is a measuring tray to bear a detection object such as the caster Ca fitted on the lower end of the leg BLa of the bed BD, or the like. The placement part 3 primarily includes a placement plate 31, the movable slope 32 connected pivotally to the placement plate 31, a wall 33 enclosing three sides of the placement plate 31, and first and second connection plates 341 and 342 for connecting the placement part 3 to the first and second beam-type load cells 21 and 22.

In this embodiment, the placement plate 31 is a rectangular flat plate with the front/rear direction as its short side direction and with the width direction as its long side direction. On the upper surface of the placement plate 31, a recess 31r having a U-shape and opening at the front side is provided along the outer periphery of the placement plate 31. As will be described later on, an arm 32a of the movable slope 32 is accommodated in the recess 31r when the caster Ca is placed on the placement plate 31.

The movable slope 32 is a plate-like member having a slope 32s, and the arm 32a connected integrally with the slope 32s.

The slope 32s is approximately rectangular in planer view, and its upper surface defines an inclined surface (a slope) for moving the caster Ca on the placement plate 31 from the floor surface. The slope 32s is heavier in weight than the arm 32a. In side view, the slope 32s is formed in a taper-like shape such that the leading end (front end) SLT of the slope 32s (and the movable slope 32) is tapered forward.

The arm 32a has an almost U-shape in planar view, having a first arm 32a1 and a second arm 32a2 connected to the two opposite ends of the slope 32s in the width direction, and a third arm 32a3 connected to the first arm 32a1 and the second arm 32a2 at the rear side of the first and second arms 32a1 and 32a2.

Any method is applicable for providing the movable slope 32 pivotally on the placement plate 31. In this embodiment, the movable slope 32 is pivotally connected to the placement plate 31 and the wall 33 with the axis X as the center, by fitting bosses b provided on the two opposite sides of the movable slope 32 in the width direction into recessed holes c provided in the inner surfaces of aftermentioned first and second side walls 331 and 332.

Because the slope 32s is heavier in weight than the arm 32a, if no external force is applied, then the leading end SLT of the slope 32s is in contact with the floor surface on which the load detector 100a is placed, such that the caster Ca can readily ride on the slope 32s. On the other hand, after the caster Ca of the bed BD passes over the slope 32s to be guided onto the placement plate 31, the caster Ca presses the arm 32a to move the same into the recess 31r. By virtue of this, the leading end SLT of the slope 32s moves to a position apart from the floor surface (a position exerting no influence on the load detection).

The wall 33 includes the first and second side walls 331 and 332 provided on the two opposite sides of the placement plate 31 in the width direction, and a rear wall 333 provided on the rear side of the placement plate 31.

The first side wall 331 stands upward from the placement plate 31 along an edge of the placement plate 31 at one side in the width direction, while the second side wall 332 stands upward from the placement plate 31 along another edge of the placement plate 31 at the other side in the width direction. The rear wall 333 stands upward from the placement plate 31 along the rear edge of the placement plate 31. The front ends of the first side wall 331 and the second side wall 332 project beyond the front edge of the placement plate 31.

In the frontal vicinity of the first side wall 331, the first connection plate 341 is provided for connecting the placement part 3 to the first beam-type load cell 21. The first connection plate 341 is a flat plate parallel to the placement plate 31, projecting outward from the lower edge of the first side wall 331 (to the other side than the placement plate 31). The upper surface of the first connection plate 341 is a first connection part C1.

On the rear surface of the rear wall 333, the second connection plate 342 is provided for connecting the placement part 3 to the second beam-type load cell 22. In this embodiment, the second connection plate 342 has a rectangular part 342a being parallel to the placement plate 31 and projecting rearward from the lower edge of the rear wall 333 (to the other side than the placement plate 31), and a square part 342b being parallel to the rectangular part 342a and projecting from the short side of the rectangular part 342a on the side of the second side wall 332. The upper surface of the square part 342b is a second connection part C2.

The placement part 3 is connected to the first and second beam-type load cells 21 and 22 by fixing the first connection part C1 on the lower surface of the flexure element 21s of the first beam-type load cell 21 in the vicinity of the free end 21s2 and fixing the second connection part C2 on the lower surface of the flexure element 22s of the second beam-type load cell 22 in the vicinity of the free end 22s2. By virtue of this, the placement part 3 is supported by the first and second beam-type load cells 21 and 22 in a vertically slightly movable (displaceable) manner.

The damper members 41 and 42 are, respectively, elastic members having an identical shape in planar view to the flat plates 11a and 12a of the first and second bases 11 and 12. The damper members 41 and 42 are fitted on the lower surfaces of the flat plates 11a and 12a by any means such as with a double-coated tape, adhesive, or the like.

The damper members 41 and 42 are, for example, rubber sheets having a damping property in particular, but not limited thereto. It is possible to use any desirable material having a damping property (a desirable damping material) as the damper members 41 and 42. Further, a number of types of layered damping materials different in hardness may be used as the damper member 41 and/or the damper member 42.

Because the load signal having a frequency primarily from 0 to 100 Hz is needed for the biological state monitoring system 1000, it is desirable to select a damping material preferably blocking vibration at the frequency in this range, and it is more desirable to select a damping material preferably blocking vibration at the frequency ranging from 0 to 40 Hz.

The thickness of the damper members 41 and 42 may be determined on the basis of the type (performance and hardness) of the damping material in use, the weight of the subject S, and the like.

[Method for monitoring the biological state]

Next, an explanation will be made on a method for monitoring the biological state of the subject S on the bed by using the biological state monitoring system 1000.

When the biological state monitoring system 1000 is set up for the bed BD, first, the load detectors 100a to 100d are set on the floor surface, and the casters Ca to Cd, at the lower ends of the legs BLa to BLd of the bed BD, are placed respectively on the placement plates 31 of the load detectors 100a to 100d.

The placement of the load detectors 100a to 100d onto the floor surface is carried out with a tight attachment (tight contact) of the lower surfaces of the damper members 41 and 42 to the floor surface. It is possible to readily carry out the placement of the casters Ca to Cd onto the placement plates 31 via the slopes 32s of the movable slopes 32. By placing the bed BD onto the placement plates 31 of the load detectors 100a to 100d, the damper members 41 and 42 of the load detectors 100a to 100d are slightly pressed by the load of the bed BD. In this state, the lower surfaces of the damper members 41 and 42 are still attached tightly to the floor surface (in tight contact with the floor surface). Further, the first and second bases 11 and 12 of the load detectors 100a to 100d stay apart from the floor surface as much as the thickness of the damper members 41 and 42.

Thereafter, the subject S is moved onto the bed BD. By moving the subject S onto the bed BD, the damper members 41 and 42 of the load detectors 100a to 100d are further slightly pressed by the load of the subject S. In this state, the lower surfaces of the damper members 41 and 42 are still attached tightly to the floor surface. Further, the first and second bases 11 and 12 of the load detectors 100a to 100d stay apart from the floor surface as much as the thickness of the damper members 41 and 42.

Monitor of the biological state of the subject S using the biological state monitoring system 1000 primarily includes, as shown in the flow chart of Fig. 5, a load detecting step S1 for detecting the load of the subject S, a center of gravity position calculating step S2 for calculating the position of the center of gravity of the subject S on the basis of variation of the detected load of the subject S, a biological state determining step S3 for determining the biological state of the subject S on the basis of the calculated position of the center of gravity of the subject S, and a display step S4 for displaying the determined biological state.

In the load detecting step S1, the load detectors 100a, 100b, 100c, and 100d are used to detect the load of the subject S on the bed BD. Because the load detectors 100a, 100b, 100c, and 100d are arranged respectively under the legs BLa, BLb, BLc, and BLd of the bed BD, the load on the upper surface of the bed BD is detected dispersively in the four load detectors 100a, 100b, 100c, and 100d. Especially, if the center of the load (the center of gravity) is present at the center O of the bed BD, then the load is detected uniformly and dispersively in the four load detectors 100a, 100b, 100c, and 100d.

Each of the load detectors 100a, 100b, 100c, and 100d detects the load (a variation in load), and outputs the result as an analog signal to the AID converting unit 200. The A/D converting unit 200 converts the analog signal into a digital signal through a sampling period of 5 milliseconds, for example. Then, the digital signal (to be referred to below as "load signal") is outputted to the control unit 300. Hereinafter, the term "load signals sa, sb, sc, and sd" will be used to refer respectively to the load signals outputted from the load detectors 100a, 100b, 100c, and 100d, and converted into the digital form in the A/D converting unit 200.

In the center of gravity position calculating step S2, the center of gravity position calculating unit 301 calculates a position G(X, Y) of the center of gravity G of the subject S on the bed BD at a predetermined period T (equal to the above sampling period of 5 milliseconds, for example), on the basis of the load signals sa to sd from the load detectors 100a to 100d, so as to find a temporal variation in the position of the center of gravity G of the subject S (a center of gravity locus GT). Here, (X, Y) shows the coordinates on the X-Ycoordinate plane (Fig. 2) with the center O of the bed BD as the origin, taking X as the breadthwise direction and Yas the lengthwise direction.

The center of gravity position calculating unit 301 calculates the position G(X, Y) of the center of gravity G according to the following computation. That is, the G(X, Y) is calculated according to the following formulas, letting the coordinates of the load detectors 100a, 100b, 100c, and 100d be, respectively, $(X_a, Y_a)$, $(X_b, Y_b)$, $(X_c, Y_c)$, and $(X_d, Y_d)$; letting the load detection values from the load detectors 100a, 100b, 100c, and 100d be, respectively, $W_a$, $W_b$, $W_c$, and $W_d$.

[Formula 1]

$$X = \frac{X_a \times W_a + X_b \times W_b + X_c \times W_c + X_d \times W_d}{W_a + W_b + W_c + W_d}$$

[Formula 2]

$$Y = \frac{Y_a \times W_a + Y_b \times W_b + Y_c \times W_c + Y_d \times W_d}{W_a + W_b + W_c + W_d}$$

The center of gravity position calculating unit 301 calculates the position G(X, Y) of the center of gravity G through the predetermined period T on the basis of the above formulas 1 and 2, while finding the temporal variation of the position G(X, Y) of the center of gravity G, that is, the center of gravity locus GT. Then, it causes the storage unit 400, for example, to store the result.

The biological state determining unit 302 determines the biological state of the subject S on the basis of the center of gravity locus GT. The biological state determining unit 302 can determine various biological states on the basis of the position of the center of gravity G, the center of gravity locus GT, and the like. Specific examples are given as follows.

(1) Determination of the respiratory rate

As is described in Patent Literature 3, the respiration of human is performed by moving the chest and the diaphragm to expand and shrink the lungs. In this context, when the air is inhaled, i.e., when the lungs are expanded, the diaphragm is lowered downwardly, and the internal organs are also moved downwardly. On the other hand, when the air is expired, i.e., when the lungs are shrunk, the diaphragm is raised upwardly, and the internal organs are also moved upwardly. The center of gravity G moves in accordance with this movement of the internal organs, and the movement of the center of gravity G occurs approximately along the extending direction of the backbone (body axis direction). Hereinbelow, the locus of the center of gravity G which vibrates in the body axis direction in accordance with the human respiration will be referred to as "respiratory vibration locus".

The biological state determining unit 302 calculates the number of respirations of the subject S per unit time (i.e., the respiratory rate) on the basis of the respiratory vibration locus included in the center of gravity locus GT calculated by the center of gravity position calculating unit 301. In particular, for example, the respiratory rate of the subject S is found on the basis of the number of extremal points of the respiratory vibration locus.

Note that as is described in Patent Literature 3, it is possible to extract the respiratory vibration locus by eliminating, from the center of gravity locus GT of the subject S, the locus of the center of gravity movement caused by the large body motion such as the turning over (tossing and turning) or the like of the subject S, and the locus of the center of gravity movement caused by the small body motion such as the movement of hands and feet or the like of the subject S. By extracting the respiratory vibration locus with such a method, it is possible to raise the precision of calculating the respiratory rate.

(2) Determination of the body axis direction and the head position

Based on the vibration direction of the respiratory vibration locus, it is possible to determine the extending direction of the body axis of the subject S. Further, as is described in Patent Literature 4, in the body axis direction of the subject S, the load value according to the respiration of the subject S is different in the variation aspect between the load detectors present at the head side from the center of gravity G of the subject S, and the load detectors present at the feet side from the center of gravity G of the subject S. Based on this feature, it is possible to determine at which side of the center of gravity G the head of the subject S is present in the determined body axis direction.

By determining the extending direction of the body axis and the position of the head of the subject S, it is possible to estimate the posture and the like of the subject S.

(3) Determination of the heart rate

The number of human heart beats, i.e., the heart rate, is 30 to 200 times or so per minute, and thus its period is approximately 0.3 to 2.0 seconds (its frequency is approximately 0.5 to 3.3 Hz). Therefore, if only the components vibrating at approximately 0.5 to 3.3 Hz are taken out from the load signals sa to sd from the load detecting unit 100 by way of frequency separation, to calculate the center of gravity position with the formulas 1 and 2 using those components, then the found position of the center of gravity (to be referred to below as "center of gravity of heart rate Gh") vibrates according to the heart rate of the subject S.

After the center of gravity position calculating unit 301 is caused to calculate the center of gravity of heart rate Gh in the above manner, it is possible for the biological state determining unit 302 to find the heart rate of the subject S on the basis of the number of extremal points present on the locus of the center of gravity of heart rate Gh.

[Measuring compensation due to the damper members]

Next, an explanation will be made on measuring compensation due to the damper members 41 and 42 in the load detectors 100a to 100d of this embodiment.

In the case of detecting the load of the subject on the bed with the four load detectors arranged to support the bed at four points, it is required theoretically the following points, for reflecting the state of the subject more correctly in the detection values of the four load detectors with the load of the subject being appropriately applied to the four load detectors. That is, the four supported points of the bed be on a single plane (condition 1), and the upper surfaces of the placement plates of the four load detectors be on a single plane (condition 2). In such case, the four supported points of the bed (the lower ends of the four casters, for example), and the four placement plates are respectively in a constantly stable contact state (four-point supported state). Then, the load on the bed BD is applied to the four load detectors via the four supported points, under an appropriate distribution according to the positional relation between the subject and the four supported points.

Conversely, if the condition 1 and/or the condition 2 are/is not satisfied, then the bed BD falls into a three-point supported state, such that more load is applied to the three load detectors involved in the three-point support, thereby lessening the load application to the other one load detector. As a result, the balance changes in the load values applied to the four load detectors, causing a variation in the detection values from the four load detectors, too. It is difficult to acquire the biological state of the subject at a high precision, based on the detection values including such a variation.

Here, in order to more correctly reflect the state of the subject in the detection values of the four load detectors, as an example, it is desirable to let the four supported points of the bed and the upper surfaces of the placement plates of the four load detectors be within a plane having a flatness not exceeding the displacement range of the measuring trays of the load detectors (for example, about 0.1 mm). However, it is not easy to satisfy this condition with beds available in the market and on a floor surface in an ordinary building.

The load detectors 100a to 100d of this embodiment are intended to realize such a state that the four supported points of a bed may be in a stabilized contact with the four placement plates 31 by using the damper members 41 and 42, without satisfying the condition 1 and the condition 2.

That is, the load detectors 100a to 100d of this embodiment are configured to change the thickness of the damper members 41 and 42 according to the load applied from the bed, so as to absorb the deviation from the plane of the supported points of the bed and/ or from the plane of the placement plates 31, so as to keep the state where the four supported points of the bed are in the stabilized contact with the four placement plates 31.

Therefore, if the load detectors 100a to 100d of this embodiment are used, then it is possible to restrain the bed BD from falling into the three-point supported state, so as to apply the load on the bed BD to the four load detectors in an appropriate distribution according to the positional relation between the subject and the four supported points via the four supported points (for example, the lower surfaces of the four casters Ca to Cd). Thus, it is possible to detect the load of the subject S at a higher precision.

Further, the floor surfaces on which the load detectors 100a to 100d are placed are not limited to being completely static. For example, there are slight vibrations due to walks of people on the floor surface, and influences of the devices placed on the floor surface causing slight vibrations (such as refrigerators, electric fans and the like). Further, the floor surface may also vibrate along with vibrations of the entire building including the floor surface in question due to powerful wind or the passages of large vehicles. With the damper members 41 and 42, the load detectors 100a to 100d of this embodiment can reduce the influence of such slight vibrations of the floor surface on the load detection.

The biological state monitoring system 1000 of this embodiment and the load detectors 100a to 100d have such effects as follows.

The load detectors 100a to 100d of this embodiment can keep the state where the lower ends of the four casters Ca to Cd are in the stabilized contact with the placement plates 31 of the four load detectors 100a to 100d, by changing the thickness of the damper members 41 and 42. That is, it is possible to restrain the bed BD from falling into the three-point supported state.

Therefore, if the load detectors 100a to 100d of this embodiment are used, then it is possible to apply the load of the subject S on the bed BD to the load detectors 100a to 100d in a more appropriate distribution via the four casters Ca to Cd. Thus, it is possible to detect the load of the subject S at a higher precision.

Because the load detectors 100a to 100d of this embodiment have the damper members 41 and 42, it is possible to reduce the influence on the load detection from the slight vibrations of the floor surface on which the load detectors 100a to 100d are placed. Thus, it is possible to detect the load of the subject S at a higher precision.

The biological state monitoring system 1000 of this embodiment can obtain the position of the center of gravity of the subject S, the biological state and the like of the subject S at a high precision, based on the load values detected by the load detectors 100a to 100d at a high precision.

Further, it is possible to attain the effect of restraining the three-point supported state from arising and the effect of reducing the influence from the vibration of the floor surface at a low cost and with a simple configuration at the same time. This is also an advantageous effect of the load detectors 100a to 100d and the biological state monitoring system 1000 of this embodiment.

That is, if restraining the three-point supported state from arising is only purpose, then it is conceivable to provide an adjuster, for adjusting the height, for the legs of the bed and for the load detectors, thereby realizing the four-point support by bringing the casters in tight contact with the placement plates. However, such an approach requires for sophisticated workers and, nevertheless, still cannot obtain the effect of reducing the influence from the floor surface vibration.

Further, there are various aspects of occurrence of the three-point supported state, and thus it is difficult to predict which of the four legs will be in unstable contact with the load detector. Further, an unstable state of contact with the load detector may change from one leg to another because of some motion of the subject on the bed. Therefore, it is not easy to eliminate errors arising from the three-point supported state through a filter process.

On the other hand, if it suffices to reduce the influence of the floor surface vibration only, then because the influence of the floor surface vibration is exerted on the four load sensors synchronously, it is not difficult to specify and then eliminate the same through a filter process. However, such an approach not only leads to complication of the system and to a high cost but also cannot restrain the three-point supported state from arising.

On the other hand, provided only with the damper members 41 and 42 on the lower surfaces of the first and second bases 11 and 12, the load detectors 100a to 100d of this embodiment can attain the effect of restraining the three-point supported state from arising and the effect of reducing the influence from the vibration of the floor surface at a low cost and with a simple configuration at the same time. Further, there is no need for sophisticated workers for restraining the three-point supported state from arising.

<Comparative examination>

Next, an explanation will be made on a comparative examination carried out by using the load detectors 100a to 100d of this embodiment, and load detectors 100A to 100D of a comparative example. The load detectors 100 A to 100D in the comparative example have the same configuration as the load detectors 100a to 100d except that there are no damper members 41 and 42.

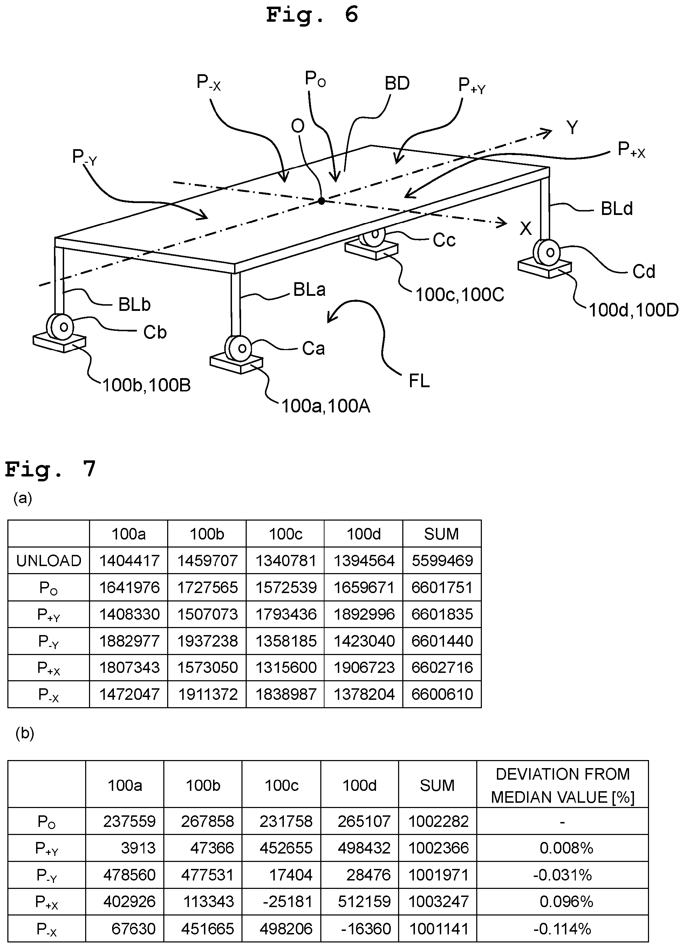

The comparative examination was carried out according to the following procedure. First, as shown in Fig. 6, under the casters Ca to Cd at the lower ends of the four legs BLa to BLd of the bed BD, the load detectors 100a to 100d of this embodiment were provided respectively. Next, weight (deadweight) having a predetermined weight was placed in the following order: at the center O of the bed BD (position $P_O$), at a position on the positive side along the axis Yfrom the center O (position $P_{+Y}$), at a position on the negative side along the axis Yfrom the center O (position $P_{-Y}$), at a position on the positive side along the axis X from the center O (position $P_{+x}$), and at a position on the negative side along the axis X from the center O (position $P_{-x}$). Then, the detection values (AD values) of the load detectors 100a to 100d were recorded with the weights being placed at the respective positions.

Thereafter, the load detectors 100a to 100d under the casters Ca to Cd of the bed BD were replaced with the load detectors 100A to 100D of the comparative example, and the same weight as used for the load detectors 100a to 100d was placed in order at the position $P_O$, at the position $P_{+Y}$, at the position $P_{-Y}$, at the position $P_{+x}$, and at the position $P_{-x}$. Then, the detection values (AD values) of the load detectors 100A to 100D were recorded with the weight being placed at the respective positions.

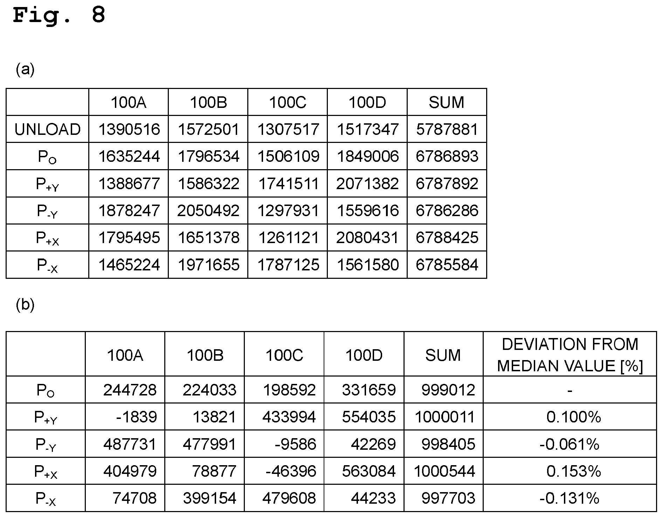

Fig. 7(a) is a table organizing the detection values of the load detectors 100a to 100d, and the sum of the detection values of the load detectors 100a to 100d, in an unloaded state without the weight being placed on the bed BD, and in each of states in which the weight is placed on the bed BD at the position $P_O$, at the position $P_{+Y}$, at the position $P_{-Y}$, at the position $P_{+x}$, and at the position $P_{-x}$. Fig. 8(a) is a table organizing the detection values of the load detectors 100A to 100D, and the sum of the detection values of the load detectors 100A to 100D, in an unloaded state without the weight being placed on the bed BD, and in each of states in which the weight is placed on the bed BD at the

position $P_0$, at the position $P_{+Y}$, at the position $P_{-Y}$, at the position $P_{+x}$, and at the position $P_{-x}$.

Fig. 7(b) is a table organizing the values of subtracting the detection values of the load detectors 100a to 100d in the unloaded state without the weight being placed on the bed BD (that is, the values corresponding to the weight of the bed BD; the values shown in the first line of Fig. 7(a)), from the respective detection values of the load detectors 100a to 100d and in each of states in which the weight is placed on the bed BD at the position $P_0$, at the position $P_{+Y}$, at the position $P_{-Y}$, at the position $P_{+x}$, and at the position $P_{-x}$ (that is, the values shown from the second lines to the sixth lines of Fig. 7(a)). Fig. 8(b) is a table organizing the values of subtracting the detection values of the load detectors 100A to 100D in the unloaded state without the weight being placed on the bed BD (that is, the values corresponding to the weight of the bed BD; the values shown in the first line of Fig. 8(a)), from the respective detection values of the load detectors 100A to 100D and in each of states in which the weight is placed on the bed BD at the position $P_0$, at the position $P_{+Y}$, at the position $P_{-Y}$, at the position $P_{+x}$, and at the position $P_{-x}$ (that is, the values shown from the second lines to the sixth lines of Fig. 8(a)). That is, the values of the tables of Figs. 7(b) and 8(b) correspond to the weight of the weight (deadweight) in the detection values of the load detectors 100a to 100d and 100 A to 100D.

The following results was confirmed by the tables of Figs. 7(a) and 7(b) through Figs. 8(a) and 8(b).

(1) In the table of Fig. 8(a) showing the result when the load detectors 100A to 100D are used, the value of the "unloaded" case is 1,572,501 at the most in the load detector 100B, and 1,307,517 at the least in the load detector 100C. The difference is 264,988. On the other hand, in the table of Fig. 7(a) showing the result when the load detectors 100a to 100d are used, the value of the "unloaded" case is 1,459,707 at the most in the load detector 100b, and 1,340,78 1 at the least in the load detector 100c. The difference is 118,926.

In the above manner, it can be said that the load from the bed is applicable more equally or evenly to the four load detectors when the load detectors 100a to 100d are used than when the load detectors 100A to 100D are used (that is, the inclination to the three-point support is suppressed so as to attain a state closer to the ideal four-point support).

(2) In the table of Fig. 8(b), the detection value of the load detector 100A is negative when the weight is placed at the position $P_{+Y}$. This means that, as a result of placing the weight on the bed BD at the position $P_{+Y}$, the bed BD is primarily supported by the casters Cb, Cc, and Cd, thereby reaching into the three-point supported state, and thus, the load from the caster Ca is not appropriately applied to the load detector 100A.

In the table of Fig. 8(b), the detection value of the load detector 100C is also negative when the weight is placed at the positions $P_{-Y}$ and $P_{+x}$. It is understood that in this state, the bed BD has also reached into the three-point supported state.

In the above manner, when the load detectors 100A to 100D of the comparative example are used, if the weight is placed at the position $P_{+Y}$, $P_{-Y}$ or $P_{+x}$, then the bed BD reaches into the three-point supported state.

On the other hand, in the table of Fig. 7(b), when the weight is placed at the position $P_{+x}$ and $P_{-x}$, the detection values of the load detector 100c and 100d are negative, respectively, whereas when the weight is placed at the positions $P_{+Y}$ or $P_{-Y}$, the detection values of the load detectors 100a to 100d are all positive. That is, if the load detectors 100a to 100d of the embodiment are used, then the bed BD reaches into the three-point supported state only in the case of placing the weight at the position $P_{+Y}$ or $P_{-Y}$, such that the three-point supported state is restrained from arising in comparison with the case of using the load detectors 100A to 100D of the comparative example.

Note that by adjusting the type and the thickness of the damper members 41 and 42, it is possible to preferably restrain the three-point supported state from arising.

(3) The rightmost columns in the table of Fig. 7(b) shows the deviation (%) of the sum of the detection value of the load detectors 100a to 100d when the weight is placed at each of position $P_{+Y}$, position $P_{-Y}$, position $P_{+x}$, and position $P_{-x}$, from the sum (median value) of the detection values of the load detectors 100a to 100d when the weight is placed at the position $P_0$. The rightmost columns in the table of Fig. 8(b) shows the deviation (%) of the sum of the detection value of the load detectors 100A to 100D when the weight is placed at each of position $P_{+Y}$, position $P_{-Y}$, position $P_{+x}$, and position $P_{-x}$, from the sum (median value) of the detection values of the load detectors 100A to 100D when the weight is placed at the position $P_0$. These deviation values show the ratio of change in the sum of the detection values by the four load detectors when the weight moves from the center O of the bed BD to the positive and negative sides in the axis-Ydirection and to the positive and negative sides in the axis-X direction. It can be said that the smaller the values, the smaller the amount of the measuring error caused by the bed BD reaching into the three-point supported state.

According to the table of Fig. 8(b), the deviation is 0.100% when the weight moves to the position $P_{+Y}$; the deviation is -0.061% when the weight moves to the position $P_{-Y}$; the deviation is 0.153% when the weight moves to the position $P_{+x}$; and the deviation is -0.131% when the weight moves to the position $P_{-x}$. Subtraction of the minimal value -0.131% from the maximal value 0.153% amounts to 0.284%. This means that, measuring error to such an extent may be caused in the detection value of the load, due to the bed BD reaching into the three-point supported state according to the movement of the load applied to the bed BD.

On the other hand, according to the table of Fig. 7(b), the deviation is 0.008 % when the weight moves to the position $P_{+Y}$; the deviation is -0.031% when the weight moves to the position $P_{-Y}$; the deviation is 0.096% when the weight moves to the position $P_{+X}$; and the deviation is -0.114% when the weight moves to the position $P_{-X}$. In each case, the value is smaller than the value in Fig. 8(b). Subtraction of the minimal value -0.114% from the maximal value 0.096% amounts to 0.210%, which is also smaller than the value in Fig. 8(b).

That is, when the load detectors 100a to 100d of the embodiment are used, the measuring error due to the bed BD reaching into the three-point supported state is smaller than when the load detectors 100 A to 100D of the comparative example are used, such that the three-point supported state is restrained from arising (the extent of occurrence is suppressed or the inclination to the three-point supported state is suppressed).

Note that it is possible to further lessen the measuring error by adjusting the type and the thickness of the damper members 41 and 42.

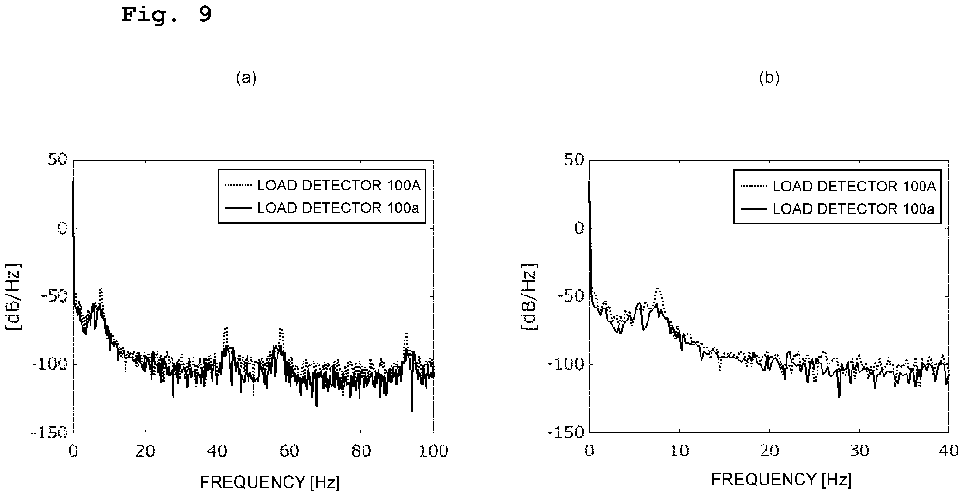

Further, investigation was made on the influence on the detection value when a vibration of 0 Hz to 100 Hz was applied to the floor surface, with the load detector 100a of the embodiment and the load detector 100A of the comparative example being placed on the floor surface. From Figs. 9(a) and 9(b), it is understood that over almost the entire range of 0 Hz to 100 Hz, the vibration is more preferably decayed in the load detector 100a having the damper members 41 and 42 than in the load detector 100A not having the damper members 41 and 42. That is, it can be said that the load detector 100a having the damper members 41 and 42 is less likely to be affected by the floor surface vibration than the load detector 100A not having the damper members 41 and 42.

<Modified embodiments>

It is possible to adopt the following modifications for the biological state monitoring system 1000 of the above embodiment.

In the load detectors 100a to 100d included in the biological state monitoring system 1000 of the above embodiment, on the lower surfaces of the flat plates 11a and 12a of the first and second bases 11 and 12, the damper members 41 and 42, which are identical respectively to the flat plates 11a and 12a in the shape according to planar view, are fitted. However, the present invention is not limited to this.

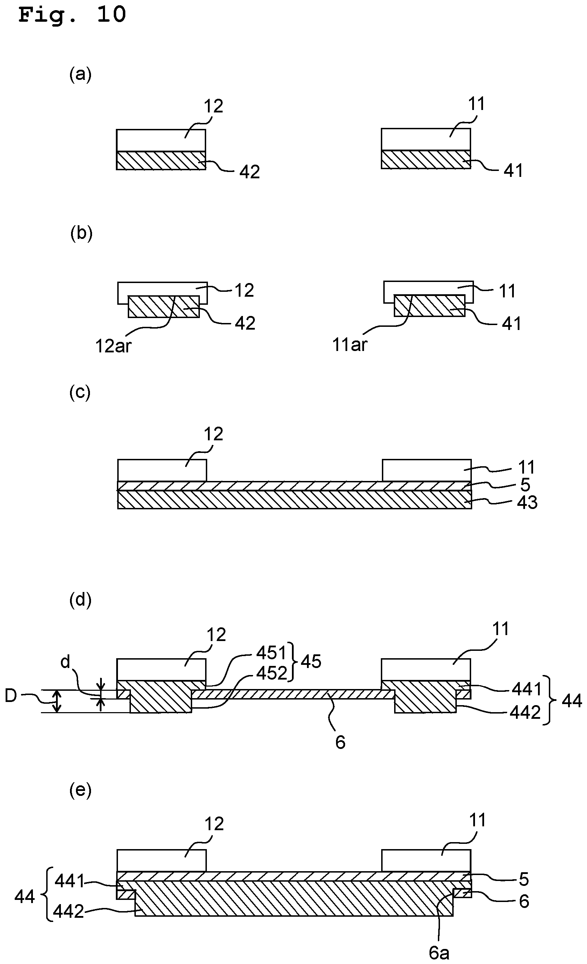

As a first modified embodiment, on the lower surfaces of the flat plates 11a and 12a, recesses 11ar and 12ar, which are one size smaller than the flat plates 11a and 12a in shape according to planar view, may be provided; and the damper members 41 and 42, which are almost identical to the recesses 11ar and 12ar in shape according to planar view, be fitted in such a manner as with only parts of the damper members 41 and 42 in the vicinity of the upper surfaces being embedded in the recesses 11ar and 12ar (Fig. 10(b)). According to this configuration, it is possible to preferably prevent the damper members 41 and 42 from deviating from the first and second bases 11 and 12.

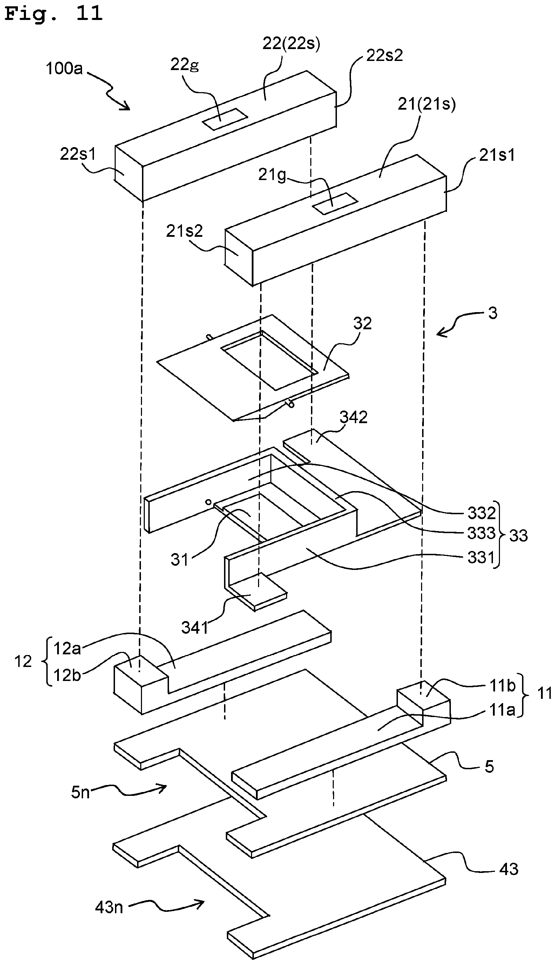

As a second modified embodiment, as shown in Fig. 11 and Fig. 10(c), below the first and second bases 11 and 12, a plate 5 may be fitted across the first base 11 and the second base 12 and, on the lower surface of the plate 5, a damper member 43 be fitted.

The plate 5 and the damper member 43 are approximately square and, in the front edge, notches 5n and 43n are provided so as not to impede the leading end SLT of the movable slope 32 of the placement part 3 from contact with the floor surface. The plate 5 is fitted on the lower surfaces of the flat plates 11a and 12a of the first and second bases 11 and 12, and the damper member 43 is fitted on the lower surface of the plate 5, respectively, by any suitable way such as with a double-coated tape, adhesive, screws, or the like.

The plate 5 may be metallic for example, but is not limited to that. Although the plate 5 may be set at any thickness, it is desirable to have such a thickness as not to give rise to flexure in the plate 5 in load detection. The material and thickness of the damper member 43 may be selected appropriately in the same manner as in the above embodiment.

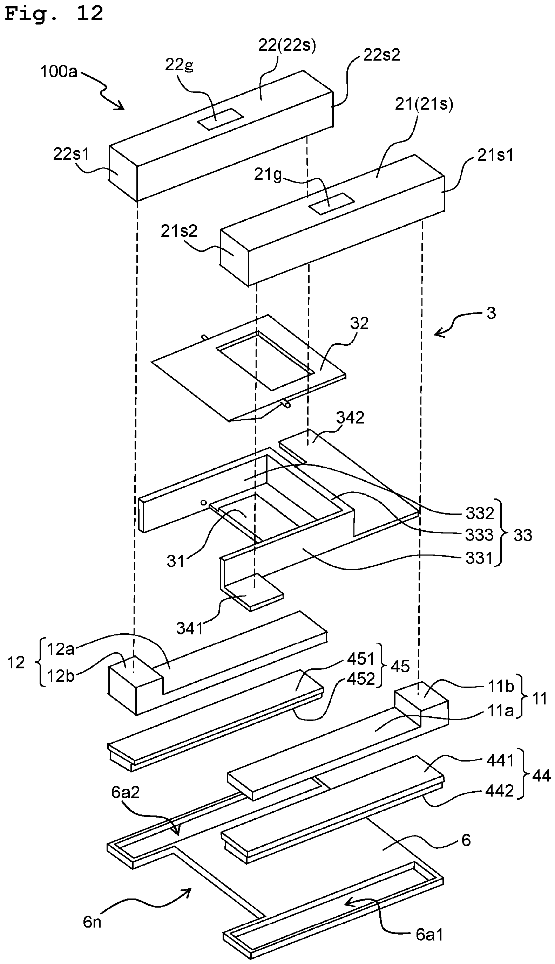

As a third modified embodiment, as shown in Fig. 12 and Fig. 10(d), damper members 44 and 45 may be fitted on the lower surfaces of the flat plates 11a and 12a of the first and second bases 11 and 12 by sandwiching the damper members 44 and 45 between the first and second bases 11 and 12 and a cover 6.

The damper members 44 and 45 have, respectively, first parts 441 and 451 being almost equal to the flat plates 11a and 12a in shape and size according to planar view, and second parts 442 and 452 being one size smaller than the first parts 441 and 451 according to planar view.

The cover 6 is a member for sandwiching the damper members 44 and 45 between itself and the first and second bases 11 and 12 so as to fix and retain the same. The cover 6 is approximately square in planar view and, in the front edge, a notch 6n is provided so as not to impede the leading end SLT of the movable slope 32 of the placement part 3 from contact with the floor surface. Further, at the two opposite ends of the cover 6 in the width direction, there are provided openings 6a1 and 6a2 almost equal to the second parts 442 and 452 of the damper members 44 and 45 in shape and size according to planar view. The cover 6 may be made of resin for example, but is not limited to that.

The cover 6 is fitted on the first and second bases 11 and 12, with the lower surfaces of the first parts 441 and 451 of the damper members 44 and 45 being in tight contact with the upper surface of the cover 6 and with the second parts 442 and 452 of the damper members 44 and 45 projecting downward through the openings 6a1 and 6a2 of the cover 6. It is possible to fit the cover 6 onto the first and second bases 11 and 12 by, for example, hooking ancyroid engagement claws (not shown) extending upward from the circumferential edge of the cover 6 onto the first and second bases 11 and 12.

Further, as shown in Fig. 10(d), let D be the thickness of the second parts 442 and 452 of the damper members 44 and 45, and let d be the thickness of the cover 6. Then, it is desirable to design for the value of D - d to be larger than zero even with the maximal load being applied on the bed BD. By virtue of this, it is possible to prevent the cover 6 from contact with the floor surface and prevent the vibration of the floor surface from direct transmission to the first and second beam-type load cells 21 and 22 without transmitting through the damper members 44 and 45.

As a fourth modified embodiment, a combination of the second modified embodiment and the third modified embodiment may be used according to certain aspects (Fig. 10(e)).

The load detector 100a of the fourth modified embodiment has the plate 5 being approximately square in planar view and fitted on the lower surfaces of the flat plates 11a and 12a of the first and second bases 11 and 12, the cover 6 being approximately square in planar view and having an approximately square opening in planar view at the center, and the damper member 44 being approximately rectangular in planar view and sandwiched by the first and second bases 11 and 12 and the cover 6. The second part 442 of the damper member 44 projects downward below the cover 6 through the opening 6a of the cover 6.

The load detectors 100a to 100d included in the biological state monitoring system 1000 of the above embodiment do not necessarily have the first and second bases 11 and 12 and the first and second beam-type load cells 21 and 22 but, instead of those, may be configured to have a single base and a single beam-type load cell. Further, the load cells are not limited to a beam type. For example, a load cell obtained by attaching a strain gage on a plate-like flexure element may be used.

In the bed BD used together with the load detectors 100a to 100d or the biological state monitoring system 1000 of the above embodiment, the four legs BLa to BLd may not be necessarily located at the four corners of the bed. The load detectors 100a to 100d and the biological state monitoring system 1000 of the above embodiment can restrain the three-point supported state from arising by the same principle as in the above embodiment even if the bed BD is supported at any four points.

In the biological state monitoring system 1000 of the above embodiment, it is also possible to establish a load detecting system by replacing the control unit 300 with a simple configuration (load calculating unit) substantially having such a part only as to find the load value of the subject S on the basis of the load signals sa to sd of the center of gravity position calculating unit 301.

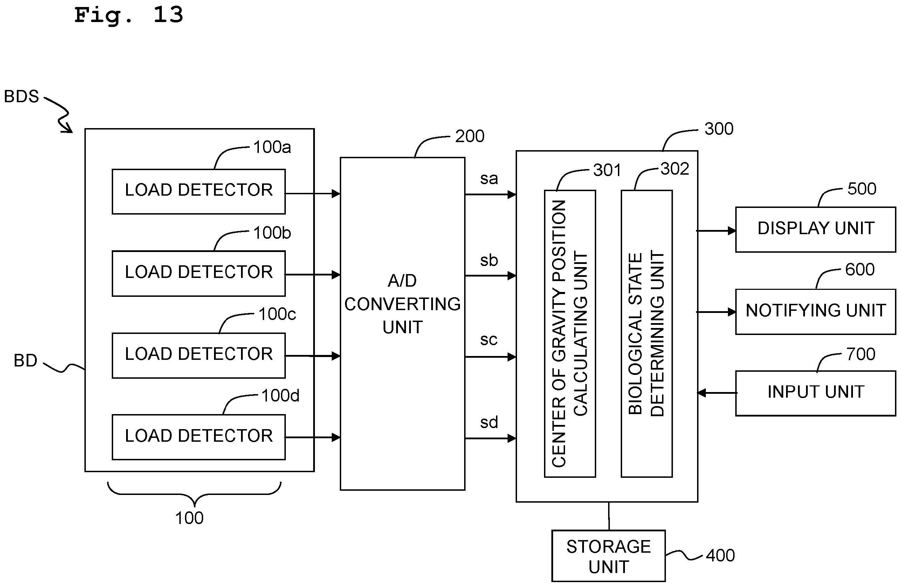

In the biological state monitoring system 1000 of the above embodiment, the load detectors 100a to 100d are arranged respectively under the casters C fitted on the lower ends of the legs of the bed BD. However, without being limited to that, the load detectors 100a to 100d may be provided between the four legs and the floor board of the bed BD and, if each of the four legs of the bed BD can be divided into upper part and lower part, then they may be provided between the upper legs and the lower legs. Further, by a combination of the load detectors 100a to 100d with the bed BD integrally or removably, it is possible to configure a bed system BDS comprised of the bed BD and the biological state monitoring system 1000 of this embodiment (Fig. 13).

In the biological state monitoring system 1000 of the above embodiment, between the load detecting unit 1 and the A/D converting unit 200, it is possible to provide a signal amplifying unit to amplify the load signal from the load detecting unit 100, and/or a filtering unit to eliminate the noises from the load signal.

In the biological state monitoring system 1000 of the above embodiment, the display unit 500 may include a simplified visible display means such as a printer for printing out information showing the biological state, a lamp displaying the biological state, and/or the like, instead of the monitor or in addition to the monitor. The notifying unit 600 may include a vibration generating unit for carrying out the notification by way of vibration, instead of the speaker or in addition to the speaker.

INDUSTRIAL APPLICABILITY

According to the biological state monitoring system of the present invention, even only with the damper members, it is still possible to restrain the three-point supported state from arising and shield the vibration from the floor surface. Therefore, it is possible to provide for hospitals and care facilities, a system capable of finding the states of hospitalized patients, care receivers in care facilities, and the like, at a higher precision and in a simple and inexpensive fashion.

PARTS LIST

11: first base, 12: second base, 21: first beam-type load cell, 22: second beam-type load cell, 3: placement part, 41, 42, 43, 44, 45: damper member, 5: plate, 6: cover, 100: load detecting unit, 100a, 100b, 100c, 100d: load detector, 200: A/D converting unit, 300: control unit, 301: center of gravity position calculating unit, 302: biological state determining unit, 400: storage unit, 500: display unit, 600: notifying unit, 700: input unit, 1000: biological state monitoring system, BD: bed, BDS: bed system, S: subject.

**Claims**

1. A load detector (100a, 100b, 100c, 100d) to be used in a biological state monitoring system (1000),

   the biological state monitoring system being configured to determine a biological state of a subject based on an output of four load detectors provided with respect to a bed (BD) so as to detect a load of the subject on the bed via four points of the bed,
   the load detector comprising:

      a base (11, 12);
      a load cell (21, 22) supported on the base in a cantilever manner to have a free end;
      a placement part (3) which is attached to the load cell on a side of the free end of the load cell and onto which the bed is to be placed; and
      a damper member (41, 42) which is provided below the base and which is a sheet having elasticity,
      wherein the damper member is provided to prevent a contact between the base and an installation surface on which the load detector is installed, in a state that the bed is placed on the placement part.

2. The load detector according to claim 1, wherein the four load detectors of the biological state monitoring system are configured to be provided at four corners of the bed.

3. The load detector according to any one of claims 1 or 2, wherein the damper member is attached to the base in contact with a lower surface of the base.

4. The load detector according to any one of claims 1 or 2, wherein the base includes a first base (11) and a second base (12);

   the load cell includes a first load cell (21) which is supported on the first base in a cantilever manner to have a free end, and a second load cell (22) which is disposed to face the first load cell and which is supported on the second base in a cantilever manner to have a free end;
   the placement part further includes a first connection part (C1) connected to the first load cell and a second connection part (C2) connected to the second load cell, the placement part being disposed between the first load cell and the second load cell;
   the free end of the first load cell and the free end of the second load cell face opposite directions to each other in a front rear direction; and
   the first connection part of the placement part is connected to the first load cell on a side of the free end of the first load cell and the second connection part of the placement part is connected to the second load cell on a side of the free end of the second load cell,
   the load detector further comprising a plate (5) attached to a lower surface of the first base and a lower surface of the second base, and
   wherein the damper member is attached to a lower surface of the plate.

5. The load detector according to any one of claims 1, 2, and 4 further comprising a cover member (6) which is provided below the base and which has an opening, and

   wherein the damper member has a first part (441, 451) having a size in plan view larger than a size in plan view of the opening, and a second part (442, 452) having a size in plan view smaller than the size in plan view of the opening, and
   the cover member and the base are configured to sandwich the first part of the damper member in a state that the second part of the damper member protrudes downwardly via the opening.

6. A load detecting system (1000) comprising:

   four load detectors (100a, 100b, 100c, 100d) according to one of the preceding claims provided with respect to a bed (BD) so as to detect a load of the subject on the bed via four points of the bed; and
   a load calculating unit (300) configured to calculate the load of the subject based on an output of the four load detectors.

7. A biological state monitoring system (1000) comprising:

   four load detectors (100a, 100b, 100c, according to any of claims 1 to 5 provided with respect to a bed (BD) so as to detect a load of the subject on the bed via four points of the bed; and
   a biological state determining unit (302) configured to determine a biological state of the subject based on an output of the four load detectors.

8. The biological state monitoring system according to claim 7 further comprising a center of gravity position calculating unit (301) configured to calculate a center of gravity position of the subject based on the output from the four load detectors, and
   wherein the biological state determining unit is configured to determine the biological state of the subject based on a temporal variation of the center of gravity position of the subject calculated by the center of gravity position calculating unit.

9. A bed system (BDS) comprising:

   a bed (BD); and
   a biological state monitoring system (1000) as defined in claim 7 or 8,
   wherein the four load detectors of the biological state monitoring system support the bed at four points.


**Patentansprüche**

1. Lastdetektor (100a, 100b, 100c, 100d) zur Verwendung in einem System zur Überwachung eines biologischen Zustands (1000),

   wobei das System zur Überwachung eines biologischen Zustands dazu konfiguriert ist, einen biologischen Zustand eines Subjekts basierend auf einer Ausgabe von vier Lastdetektoren zu bestimmen, die in Bezug auf ein Bett (BD) bereitgestellt werden, um eine Last des Subjekts auf dem Bett über vier Punkte des Betts zu erkennen,
   wobei der Lastdetektor umfasst:

   eine Basis (11, 12);
   eine Wägezelle (21, 22), die auf der Basis freitragend gelagert ist und ein freies Ende aufweist;
   ein Platzierungsteil (3), das an der Wägezelle auf einer Seite des freien Endes der Wägezelle befestigt ist, und auf welches das Bett platziert werden soll; und
   ein Dämpfungselement (41, 42), das unterhalb der Basis bereitgestellt ist und das eine Platte ist, die Elastizität aufweist,
   wobei das Dämpfungselement bereitgestellt ist, um einen Kontakt zwischen der Basis und einer Installationsfläche, auf welcher der Lastdetektor installiert ist, in einem Zustand zu verhindern, in dem das Bett auf dem Platzierungsteil platziert ist.

2. Lastdetektor nach Anspruch 1, wobei die vier Lastdetektoren des Systems zur Überwachung eines biologischen Zustands so konfiguriert sind, dass sie an vier Ecken des Bettes bereitgestellt werden.

3. Lastdetektor nach einem der Ansprüche 1 oder 2, wobei das Dämpfungselement an der Basis in Kontakt mit einer unteren Fläche der Basis befestigt ist.

4. Lastdetektor nach einem der Ansprüche 1 oder 2, wobei die Basis eine erste Basis (11) und eine zweite Basis (12) beinhaltet;

die Wägezelle eine erste Wägezelle (21) beinhaltet, die freitragend auf der ersten Basis gelagert ist und ein freies Ende aufweist, und eine zweite Wägezelle (22), die so angeordnet ist, dass sie der ersten Wägezelle gegenüberliegt, und die freitragend auf der zweiten Basis gelagert ist und ein freies Ende aufweist;

das Platzierungsteil ferner ein erstes Verbindungsteil (C1), das mit der ersten Wägezelle verbunden ist, und ein zweites Verbindungsteil (C2) beinhaltet, das mit der zweiten Wägezelle verbunden ist, wobei das Platzierungsteil zwischen der ersten Wägezelle und der zweiten Wägezelle angeordnet ist;

das freie Ende der ersten Wägezelle und das freie Ende der zweiten Wägezelle einander gegenüberliegende Richtungen in einer vorderen und hinteren Richtung aufweisen; und

das erste Verbindungsteil des Platzierungsteils mit der ersten Wägezelle auf einer Seite des freien Endes der ersten Wägezelle verbunden ist und das zweite Verbindungsteil des Platzierungsteils mit der zweiten Wägezelle auf einer Seite des freien Endes der zweiten Wägezelle verbunden ist,

der Lastdetektor ferner eine Platte (5) umfasst, die an einer unteren Fläche der ersten Basis und einer unteren Fläche der zweiten Basis befestigt ist, und

wobei das Dämpfungselement an einer unteren Fläche der Platte befestigt ist.

5. Lastdetektor nach einem der Ansprüche 1, 2 und 4, ferner umfassend ein Abdeckungselement (6), das unterhalb der Basis bereitgestellt ist und eine Öffnung aufweist, und

wobei das Dämpfungselement einen ersten Teil (441, 451), der in der Draufsicht eine Größe aufweist, die größer ist als die Größe der Öffnung in der Draufsicht, und einen zweiten Teil (442, 452) aufweist, der in der Draufsicht eine Größe aufweist, die kleiner ist als die Größe der Öffnung in der Draufsicht, und

das Abdeckungselement und die Basis so konfiguriert sind, dass sie den ersten Teil des Dämpfungselements in einem Zustand einschließen, in dem der zweite Teil des Dämpfungselements durch die Öffnung nach unten vorsteht.

6. Lastdetektionssystem (1000), umfassend:

vier Lastdetektoren (100a, 100b, 100c, 100d) nach einem der vorhergehenden Ansprüche, die in Bezug auf ein Bett (BD) bereitgestellt werden,
um eine Last des Subjekts auf dem Bett über vier Punkte des Bettes zu erkennen; und
eine Lastberechnungseinheit (300), die so konfiguriert ist, dass sie die Last des Subjekts basierend auf einer Ausgabe der vier Lastdetektoren berechnet.

7. System zur Überwachung eines biologischen Zustands (1000), umfassend:

vier Lastdetektoren (100a, 100b, 100c, nach einem der Ansprüche 1 bis 5, die in Bezug auf ein Bett (BD) bereitgestellt werden,
um eine Last des Subjekts auf dem Bett über vier Punkte des Bettes zu erkennen; und
eine Einheit zur Bestimmung eines biologischen Zustands (302), die so konfiguriert ist, dass sie ein biologisches Datum des Subjekts basierend auf einer Ausgabe der vier Lastdetektoren bestimmt.

8. System zur Überwachung eines biologischen Zustands nach Anspruch 7, ferner umfassend eine Einheit zur Berechnung der Position des Schwerpunkts (301), die so konfiguriert ist, dass sie eine Position des Schwerpunkts des Subjekts basierend auf dem Ausgangssignal der vier Lastdetektoren berechnet, und

wobei die Einheit zur Bestimmung eines biologischen Zustands so konfiguriert ist, dass sie den biologischen Zustand des Subjekts basierend auf einer zeitlichen Veränderung der Position des Schwerpunkts des Subjekts, die von der Einheit zur Berechnung der Position des Schwerpunkts berechnet wird, bestimmt.

9. Bettsystem (BDS), umfassend:

ein Bett (BD); und
ein System zur Überwachung eines biologischen Zustands (1000), wie in Anspruch 7 oder 8 definiert,
wobei die vier Lastdetektoren des Systems zur Überwachung eines biologischen Zustands das Bett an vier Punkten abstützen.

**Revendications**

1. Détecteur de charge (100a, 100b, 100c, 100d) destiné à être utilisé dans un système de surveillance d'état biologique (1000),

    le système de surveillance d'état biologique étant conçu pour déterminer un état biologique d'un sujet sur la base d'une sortie de quatre détecteurs de charge disposés par rapport à un lit (BD) de manière à détecter une charge du sujet sur le lit via quatre points du lit,
    le détecteur de charge comprenant :

        une base (11, 12) ;
        une cellule de charge (21, 22) supportée en porte-à-faux sur la base pour avoir une extrémité libre ;
        une partie de placement (3) qui est fixée à la cellule de charge sur un côté de l'extrémité libre de la cellule de charge et sur laquelle le lit doit être placé ; et
        un élément amortisseur (41, 42) qui est disposé sous la base et qui est une feuille ayant une élasticité, l'élément amortisseur permettant d'empêcher un contact entre la base et une surface d'installation sur laquelle est installé le détecteur de charge, dans un état dans lequel le lit est placé sur la partie de placement.

2. Détecteur de charge selon la revendication 1, dans lequel les quatre détecteurs de charge du système de surveillance d'état biologique sont conçus pour être disposés aux quatre coins du lit.

3. Détecteur de charge selon l'une quelconque des revendications 1 ou 2, dans lequel l'élément amortisseur est fixé à la base en contact avec une surface inférieure de la base.

4. Détecteur de charge selon l'une quelconque des revendications 1 ou 2, dans lequel la base comprend une première base (11) et une seconde base (12) ;

    la cellule de charge comprend une première cellule de charge (21) qui est supportée en porte-à-faux sur la première base pour avoir une extrémité libre, et une seconde cellule de charge (22) qui est disposée pour faire face à la première cellule de charge et qui est supportée en porte-à-faux sur la seconde base pour avoir une extrémité libre ;
    la partie de placement comprend en outre une première partie de connexion (C1) connectée à la première cellule de charge et une seconde partie de connexion (C2) connectée à la seconde cellule de charge, la partie de placement étant disposée entre la première cellule de charge et la seconde cellule de charge ;
    l'extrémité libre de la première cellule de charge et l'extrémité libre de la seconde cellule de charge sont face à des directions opposées l'une à l'autre dans une direction avant arrière ; et
    la première partie de connexion de la partie de placement est connectée à la première cellule de charge sur un côté de l'extrémité libre de la première cellule de charge et la seconde partie de connexion de la partie de placement est connectée à la seconde cellule de charge sur un côté de l'extrémité libre de la seconde cellule de charge,
    le détecteur de charge comprenant en outre une plaque (5) fixée à une surface inférieure de la première base et à une surface inférieure de la seconde base, et
    dans lequel l'élément amortisseur est fixé à une surface inférieure de la plaque.

5. Détecteur de charge selon l'une quelconque des revendications 1, 2 et 4, comprenant en outre un élément de couvercle (6) qui est disposé sous la base et qui a une ouverture, et

    dans lequel l'élément amortisseur comporte une première partie (441, 451) ayant une taille en vue en plan supérieure à une taille en vue en plan de l'ouverture, et une seconde partie (442, 452) ayant une taille en vue en plan inférieure à la taille en vue en plan de l'ouverture, et
    l'élément de couvercle et la base sont conçus pour prendre en sandwich la première partie de l'élément amortisseur dans un état dans lequel la seconde partie de l'élément amortisseur fait saillie vers le bas à travers l'ouverture.

6. Système de détection de charge (1000) comprenant :

    quatre détecteurs de charge (100a, 100b, 100c, 100d) selon l'une des revendications précédentes disposés par rapport à un lit (BD)

de manière à détecter une charge du sujet sur le lit via quatre points du lit ; et
une unité de calcul de charge (300) conçue pour calculer la charge du sujet sur la base d'une sortie des quatre détecteurs de charge.

7. Système de surveillance d'état biologique (1000) comprenant :

quatre détecteurs de charge (100a, 100b, 100c, selon l'une quelconque des revendications 1 à 5 disposés par rapport à un lit (BD)
de manière à détecter une charge du sujet sur le lit via quatre points du lit ; et
une unité de détermination d'état biologique (302) conçue pour déterminer une date biologique du sujet sur la base d'une sortie des quatre détecteurs de charge.

8. Système de surveillance d'état biologique selon la revendication 7, comprenant en outre une unité de calcul de position du centre de gravité (301) conçue pour calculer une position du centre de gravité du sujet sur la base de la sortie provenant des quatre détecteurs de charge, et
l'unité de détermination d'état biologique étant conçue pour déterminer l'état biologique du sujet sur la base d'une variation temporelle de la position du centre de gravité du sujet calculée par l'unité de calcul de position du centre de gravité.

9. Système de lit (BDS) comprenant :

un lit (BD) ; et
un système de surveillance d'état biologique (1000) tel que défini dans la revendication 7 ou 8,
dans lequel les quatre détecteurs de charge du système de surveillance d'état biologique soutiennent le lit en quatre points.

**Fig. 1**

EP 3 640 610 B1

# Fig. 2

# Fig. 3

Fig. 4

Fig. 5

| LOAD DETECTING STEP | S1 |

| CENTER OF GRAVITY POSITION CALCULATING STEP | S2 |

| BIOLOGICAL STATE DETERMINING STEP | S3 |

| DISPLAY STEP | S4 |

# Fig. 6

# Fig. 7

(a)

|  | 100a | 100b | 100c | 100d | SUM |
|---|---|---|---|---|---|
| UNLOAD | 1404417 | 1459707 | 1340781 | 1394564 | 5599469 |
| $P_O$ | 1641976 | 1727565 | 1572539 | 1659671 | 6601751 |
| $P_{+Y}$ | 1408330 | 1507073 | 1793436 | 1892996 | 6601835 |
| $P_{-Y}$ | 1882977 | 1937238 | 1358185 | 1423040 | 6601440 |
| $P_{+X}$ | 1807343 | 1573050 | 1315600 | 1906723 | 6602716 |
| $P_{-X}$ | 1472047 | 1911372 | 1838987 | 1378204 | 6600610 |

(b)

|  | 100a | 100b | 100c | 100d | SUM | DEVIATION FROM MEDIAN VALUE [%] |
|---|---|---|---|---|---|---|
| $P_O$ | 237559 | 267858 | 231758 | 265107 | 1002282 | - |
| $P_{+Y}$ | 3913 | 47366 | 452655 | 498432 | 1002366 | 0.008% |
| $P_{-Y}$ | 478560 | 477531 | 17404 | 28476 | 1001971 | -0.031% |
| $P_{+X}$ | 402926 | 113343 | -25181 | 512159 | 1003247 | 0.096% |
| $P_{-X}$ | 67630 | 451665 | 498206 | -16360 | 1001141 | -0.114% |

Fig. 8

(a)

|  | 100A | 100B | 100C | 100D | SUM |
|---|---|---|---|---|---|
| UNLOAD | 1390516 | 1572501 | 1307517 | 1517347 | 5787881 |
| $P_O$ | 1635244 | 1796534 | 1506109 | 1849006 | 6786893 |
| $P_{+Y}$ | 1388677 | 1586322 | 1741511 | 2071382 | 6787892 |
| $P_{-Y}$ | 1878247 | 2050492 | 1297931 | 1559616 | 6786286 |
| $P_{+X}$ | 1795495 | 1651378 | 1261121 | 2080431 | 6788425 |
| $P_{-X}$ | 1465224 | 1971655 | 1787125 | 1561580 | 6785584 |

(b)

|  | 100A | 100B | 100C | 100D | SUM | DEVIATION FROM MEDIAN VALUE [%] |
|---|---|---|---|---|---|---|
| $P_O$ | 244728 | 224033 | 198592 | 331659 | 999012 | - |
| $P_{+Y}$ | -1839 | 13821 | 433994 | 554035 | 1000011 | 0.100% |
| $P_{-Y}$ | 487731 | 477991 | -9586 | 42269 | 998405 | -0.061% |
| $P_{+X}$ | 404979 | 78877 | -46396 | 563084 | 1000544 | 0.153% |
| $P_{-X}$ | 74708 | 399154 | 479608 | 44233 | 997703 | -0.131% |

## Fig. 9

(a)

(b)

EP 3 640 610 B1

## Fig. 10

(a)

(b)

(c)

(d)

(e)

## Fig. 11

# Fig. 12

**Fig. 13**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4652226 B
- JP 4879620 B
- JP 6105703 B
- JP 6122188 B
- US 7282652 B1
- EP 2183554 A1
- EP 0838659 A2
- JP 2006302266 A